# EUROPÄISCHE PATENTSCHRIFT

(11) **EP 1 531 782 B1**
(45) Veröffentlichungstag und Bekanntmachung des Hinweises auf die Patenterteilung: **25.04.2007**
(21) Anmeldenummer: 03792321.6
(22) Anmeldetag: 15.08.2003
(51) Int. Cl.: A61Q 19/02, A61K 8/64

(54) **KOSMETISCHE ODER DERMATOLOGISCHE ZUBEREITUNG MIT PROTEINEN ZUR HAUTAUFHELLUNG**
COSMETIC OR DERMATOLOGICAL PREPARATION WITH SKIN-LIGHTENING PROTEINS
PREPARATION COSMETIQUE OU DERMATOLOGIQUE AUX PROTEINES POUR ECLAIRCIR LA PEAU

(30) Priorität: 21.08.2002 DE 10239029
(43) Veröffentlichungstag der Anmeldung: 25.05.2005
(73) Patentinhaber: Coty B.V., 2031 CC Haarlem (NL)
(72) Erfinder: GOLZ-BERNER, Karin, 98000 Monaco (MC); WALZEL, Bernd, CH-8003 Zürich (CH); ZASTROW, Leonhard, 98000 Monaco (MC); DOUCET, Olivier, F-06230 Villefranche s/Mer (FR)
(74) Vertreter: Walter, Wolf-Jürgen
(86) Internationale Anmeldenummer: PCT/EP2003/009046
(87) Internationale Veröffentlichungsnummer: WO 2004/017931

(56) Entgegenhaltungen:
- WO-A-98/19665
- WO-A-03/047543
- FR-A- 2 742 661
- US-A- 5 609 875
- US-B1- 6 197 304

## Beschreibung

Die Erfindung betrifft eine kosmetische oder dermatologische Zubereitung mit Proteinen oder Enzymen, die zur Aufhellung von Haut einsetzbar ist.

Aus der WO98/58628 sind zur Hautaufhellung Kojisäure, Ascorbinsäure, Bärentraubenextrakt, Maulbeerbaumextrakt, Süßholzextrakt, Arbutin und Gemische davon bekannt, die in der WO02/36090 auch in Form eines kosmetischen Pflasters angewendet werden.

In der EP 909161 werden substituierte Diphenole in einer Wasserin-Silicon-Gelzusammensetzung als Hautaufhellungsmittel beschrieben.

Die US 5 609 875 offenbart eine Kombination von Glycyrrhiza Glabra mit α- oder β-Hydroxysäuren, Ketosäureamiden oder Salzen davon zur Hautaufhellung.

Die US 6 416 756 betrifft Enzyme, die durch Polymermoleküle wie PEG modifiziert sein können und dadurch stabilisiert und im Empfindlichkeitspotenzial verringert werden können.

Der Erfindung liegt die Aufgabe zugrunde, neue kosmetische oder dermatologische Mittel zur Aufhellung menschlicher Haut bereitzustellen. Eine weitere Aufgabe besteht darin, den Hautbräunungsprozeß zu beeinflussen.

Erfindungsgemäß umfaßt die kosmetische oder dermatologische Zubereitung einen wirksamen Gehalt an modifizierten kupferbindenden Proteinen oder Enzymen, denen das Kupfer in der prosthetischen Gruppe durch eine vorherige Austauschreaktion entzogen wurde, oder an solchen modifizierten kupferbindenden-Proteiren, die durch rekombinante Verfahren ohne Kupfer hergestellt wurden.

Die Zubereitung enthält vorzugsweise solche kupferfrei-modifizierten kupferbindenden Proteine, die ausgewählt sind aus der Gruppe, bestehend aus Oxidoreduktase, Auracyanin, Azurin, Phytocyanin, Plastocyanin, Rusticyanin und Gemischen davon in ihrer modifizierten kupferfreien Form. Besonders bevorzugt sind solche aus der Gruppe der sogenannten blauen Kupfer-Proteine wie Auracyanin, Azurin, Phytocyanin, Rusticyanin, Ascorbatoxidase, Ceruloplasmin, Laccase, Nitritreduktase und Gemische davon.

Weitere bevorzugte modifizierte kupferbindende Enzyme umfassen Cu,Zn-Superoxiddismutase, Dioxygenase, Monooxygenase, Aminoxidase, Diaminooxidase, Ascorbatoxidase, Ceruloplasmin, Cytochrom-c-Oxidase, Galactoseoxidase, Lysyloxidase, Catecholoxidase, N₂O-Reduktase, Haemocyanin, Tyrosinase, Ubichinonoxidase und Gemische davon in ihrer modifizierten kupferfreien Form.

Der Gehalt der modifizierten, im wesentlichen kupferfreien Kupfer-Proteine liegt im Bereich von 0,001 bis 1 Gew-%, vorzugsweise im Bereich von 0,005 bis 0,01 Gew-%, bezogen auf das Gesamtgewicht der Zubereitung.

Es wurde gefunden, daß den Proteinen, die generell als "Kupfer-Proteine" oder "kupferbindende Proteine" bezeichnet werden und die in ihrer kupferhaltigen Form als blaue oder nichtblaue Komplexe vorliegen, die in ihrer prosthetischen Gruppe Cu-Ionen enthalten, in einer Chelatisierungsreaktion das Kupfer entzogen werden kann. Sie können auch über rekombinante Verfahren und unter Einsatz eines kupferfreien Moleküls ohne das Cu-Ion hergestellt werden. Derartige modifizierte Kupfer-Proteine sind überraschenderweise in der Lage, dem Enzym Tyrosinase, das ebenfalls ein Kupfer-Protein darstellt, in wirksamer Weise das Kupfer zu entziehen und damit die natürliche Melaninsynthese, die über die nicht-essentielle Aminosäure Tyrosin abläuft, zu unterbrechen.

Die Chelatisierungsreaktion, mit der beispielsweise das Kupfer den Kupfer-Proteinen entzogen werden kann, läuft in bekannter Weise über Kationenaustauscher oder über Chelatbildner ab, wie Polyoxycarbonsäuren, Polyamine, Ethylendiamintetraessigsäure und deren Na- oder K-Salze, Nitrilotriessigsäure usw. Derartige Reaktionen sind dem Fachmann bekannt.

Im Gegensatz zu anderen bekannten Hautaufhellern, wie z.B. Hydrochinon und Derivate davon, Azelainsäure, Kojisäure oder Arbutin, die ebenfalls die Melaninsysnthese hemmen, treten keine Nebeneffekte auf, und die modifizierten Kupferproteine sind relativ leicht über eine einfache Chelatisierungsreaktion handelsüblicher Protein- oder Enzymprodukte zugänglich. Diese können aus Pflanzenextrakten oder durch Isolierung aus Bakterien- oder Hefekulturen gewonnen werden und werden anschließend mit einem Chelatbildner behandelt.

Die Zubereitung kann zusätzlich hautaufhellende Mittel enthalten, sog. "Whitening"-Mittel, wie Bärentraubenblätterextrakt, Senfsamenextrakt von weißem Senf, Silberweidenrindenextrakt, Hibiskusblütenextrakt und Gemischen davon. Diese Extrakte können die Hautpigmentierung verringern. So enthält z.B. Bärentraubenextrakt Wirkstoffe, die die Tyrosinasebildung inhibieren. Senfsamenextrakt enthält Kampferol, das den Transfer von Melanosomen in die Keratinozyten verhindert. Extrakte der Silberweidenrinde enthalten Salicoside, die die Geschwindigkeit der Hautzellerneuerung erhöhen und damit schneller zu Hautaufhellungseffekten beitragen. Extrakte aus Hibiskusblüten haben einen hohen Gehalt an organischen Säuren, die einen keratinolytischen Effekt haben und die Entfernung von pigmentierten Hautschichten beschleunigen.

Es ist weiterhin vorteilhaft, der Zubereitung 0,1 bis 10 Gew-% eines gemahlenen, reinweißen Porzellans mit einer Teilchengröße im Bereich von 0,1 bis 100 µm zuzusetzen, wodurch in der Kombination mit den modifizierten kupferbindenden Proteinen ein besonders starker Aufhellungseffekt erreicht wird.

Es können auch wahlweise oder zusätzlich 0,1 bis 15 Gew-% reflektierende Pigmente zugesetzt werden, ausgewählt aus der Gruppe, bestehend aus gemahlenen Glaspartikeln, TiO₂, ZnO, Perlmutt, Perglanzpigmenten und Gemischen davon.

Eine weitere vorteilhafte Ausführungsform der Erfindung besteht darin, daß den kupferbindenden Proteinen gemahlene weiße Perlen mit einer Teilchengröße von 0,1-100 µm zugesetzt werden. Perlenproteine (Conchagene) ergeben zusammen mit den kupferbindenden Proteinen einen zusätzlichen und überraschend hohen Aufhellungseffekt und einen Glanzeffekt. Darüber hinaus verstärken die Conchagene die Glycoproteinverankerung in der Haut und wirken der Destabilisierung des Fasernetzwerkes des Hautkollagens entgegen, wodurch sich eine Anti-Alterungswirkung ergibt. Ein Zusatz gemahlener Perlen kann im Bereich von 0,1 bis 8 Gew-% erfolgen.

Eine weitere vorteilhafte Ausführungsform der Erfindung besteht darin, daß den kupferbindenden Proteinen ein Wurzelextrakt von Glycyrrhiza glabra zugesetzt wird oder ein Komplex, bestehend aus dem Wurzelextrakt von Glycyrrhiza glabra, dem Wurzelextrakt von Morus alba, dem Wurzelextrakt von Scutellaria baicalensis, dem Extrakt von Saxifraga stolonifera, einem Grapefruchtextrakt und Dinatriumethylendiamintetraessigsäure (EDTA). Damit wird die Hautaufhellungswirkung weiter verstärkt. Ein Zusatz von Glycyrrhiza glabra oder von dem genannten Komplex kann im Bereich von 0,1 bis 6 Gew-% erfolgen.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)prppan-1,3-dion.

Eine bevorzugte Menge an zugesetztem organischen UVA- und/oder UVB-Filter liegt im Bereich von 1 bis 20 Gew-%, vorteilhaft 1 bis 12 Gew-%, insbesondere 1 bis 3 Gew-%, bezogen auf das Gewicht der Gesamtzusammensetzung.

Die erfindungsgemäße Zubereitung enthält weiterhin kosmetische Hilfs- und Trägerstoffe, wie sie üblicherweise in solchen Zubereitungen verwendet werden, z.B. Wasser, Konservierungsmittel, Farbstoffe, Weißpigmente, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Die erfindungsgemäße Zubereitung kann weitere kosmetische Wirkstoffe enthalten. Dazu gehören z. B. Radikalfänger, Feuchthaltemittel, Vitamine, pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783; Kaolin; sowie mit SiO₂ modifiziertes Kaolin gemäß WO94/17588.

Die Zubereitung kann als Emulsion oder als Gel vorliegen. Zu bekannten geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Die für die Erfindung eingesetzten Öle können übliche kosmetische Öle sein, wie ein Mineralöl; hydriertes Polyisobuten ; synthetisches oder aus Naturprodukten hergestelltes Squalan wie Synthesqual®, Cosbiol®; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianußöl, Rizinusöl, Kakaoobutter, Kokosnußöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflußt, wie Transparenzgrad, Weichheit, Härte, Spreitungswirkung.

Als Feuchthaltemittel geeignet sind z.B. Glycerin, Butylenglycol, Propylenglycol und Gemische davon.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Die Erfindung soll nachstehend durch Beispiele näher erläutert werden. Alle Angaben erfolgen in Gewichtsprozent, sofern nichts anderes angegeben ist.

### Beispiel 1 Aufhellende und Glanz-Gesichts- und Körperlotion SPF 15

| **Phase A** | |
|---|---|
| Wasser | q.s. ad 100 |
| Glycerin | 5 |
| Timeron (Mica, TiO₂, Eisenoxide) | 1 |

| **Phase B** | |
|---|---|
| Isopropyl Palmitate | 1 |
| Tri-C12-13 Alkyl Citrate | 6 |
| C12-15 Alkyl Benzoate | 1,2 |
| Butyl Methoxydibenzoylmethane | 3 |
| Ethyl Hexyl Methoxycinnamate | 7,5 |

| **Phase C** | |
|---|---|
| RPF-Wirkstoffzubereitung* | 0,5 |
| Ascorbatoxidase (Cu-frei modifiziert) | 0,1 |
| Glycyrrhiza glabra (Licorice) | 0,2 |
| gemahlene weiße Perlen 0,5-30 µm | 0,5 |
| Hibiscusblütenextrakt | 0,1 |
| Parfümöl | 0,05 |
| Konservierungsmittel | 0,1 |

| | |
|---|---|
| * mit Quebracho blanco (2 %), Seidenraupenextrakt (1 %), trockenes Gel (1 %), Phospholipiden (7 %) und Wasser (89 %) ; Herstellung wie in WO99/66881 Beispiel 1 beschrieben. | |

Die Bestandteile der Phasen A und B werden jeweils bei etwa 75°C separat vermischt, zusammengegeben und homogenisiert. Nach dem Abkühlen auf etwa 40°C wird unter Rühren die bei dieser Temperatur aus den Einzelbestandteilen zusammengefügte Phase C zugesetzt.

Die Herstellung des modifizierten Enzyms erfolgte bei etwa 30°C mit EDTA.

### Beispiel 2 Aufhellende und Glanz-Gesichtscreme SPF 20

| **Phase A** | |
|---|---|
| Wasser | q.s. ad 100 |
| EDTA | 0,1 |
| Propylenglycol | 3 |
| Crosspolymer | 0,8 |
| Timeron (Mica, TiO₂, Eisenoxide) | 1,5 |

| **Phase B** | |
|---|---|
| Benzophenone-3 | 5 |
| Isoamyl p-Methoxycinnamate | 5 |
| Butyl Methoxydibenzoylmethane | 3 |
| Ethyl Hexyl Methoxycinnamate | 7 |

| **Phase C** | |
|---|---|
| RPF-Wirkstoffzubereitung* | 0,8 |
| Yeast extract** | 0,1 |
| Azurin (Cu-frei modifiziert) | 0,1 |
| Glycyrrhiza glabra (Licorice) | 0,5 |
| Bärentraubenblätterextrakt | 0,1 |
| Silberweidenrindenextrakt | 0,3 |
| gemahlene weiße Perlen 0,5-30 µm | 1,0 |
| Parfümöl | 0,1 |
| Konservierungsmittel | 0,1 |

| | |
|---|---|
| * mit Quebracho blanco (2 %), Seidenraupenextrakt (1 %), trockenes Gel (1 %), Phospholipiden (7 %) und Wasser (89 %) ; Herstellung wie in WO99/66881 Beispiel 1 beschrieben. ** Hefextrakt nach Beispiel 1 der DE 4241154C1 | |

Die Bestandteile der Phasen A und B werden jeweils bei etwa 75°C separat vermischt, zusammengegeben und homogenisiert. Nach dem Abkühlen auf etwa 40°C wird unter Rühren die bei dieser Temperatur aus den Einzelbestandteilen zusammengefügte Phase C zugesetzt.

Die Herstellung des modifizierten Azurins erfolgte bei etwa 28°C mit Nitrilotriessigsäure.

### Beispiel 3 Aufhellende und Glanz-Gesichtscreme SPF 20 II

| **Phase A** | |
|---|---|
| Wasser | q.s. ad 100 |
| EDTA | 0,1 |
| Propylenglycol | 3 |
| Crosspolymer | 0,8 |
| Timeron (Mica, TiO₂, Eisenoxide) | 1,5 |

| **Phase B** | |
|---|---|
| Benzophenone-3 | 5 |
| Isoamyl p-Methoxycinnamate | 5 |
| Butyl Methoxydibenzoylmethane | 3 |
| Ethyl Hexyl Methoxycinnamate | 7 |

| **Phase C** | |
|---|---|
| Wirkstoffzubereitung* | 0,8 |
| Yeast extract** | 0,1 |
| Laccase (Cu-frei modifiziert) | 0,1 |
| Komplex aus Glycyrrhiza glabra (Licorice), Morus alba, Scutellaria baicalensis, Saxifraga stolonifera, Grapefrucht-extrakt, EDTA | 1,0 |
| gemahlene weiße Perlen 0,5-30 µm | 1,0 |
| gemahlenes Porzellan 0,1-20 µm | 0,8 |
| Parfümöl | 0,1 |
| Konservierungsmittel | 0,1 |

| | |
|---|---|
| * mit Quebracho blanco (2 %), Seidenraupenextrakt (1 %), trockenes Gel (1 %), Phospholipiden (7 %) und Wasser (89 %) ; Herstellung wie in WO99/66881 Beispiel 1 beschrieben. ** Hefextrakt nach Beispiel 1 der DE 4241154C1 | |

Die Arbeitsweise entsprach der von Beispiel 2.

## Patentansprüche

1. Kosmetische oder dermatologische Zubereitung mit Proteinen zur Hautaufhellung, **dadurch gekennzeichnet, daß** die Zubereitung einen wirksamen Gehalt an kupferfrei-modifizierten kupferbindenden Proteinen oder Enzymen aufweist, denen das Kupfer in der prosthetischen Gruppe durch eine vorherige Austauschreaktion entzogen wurde oder die durch rekombinante Verfahren ohne Kupfer hergestellt wurden.

2. Zubereitung nach Anspruch 1, **dadurch gekennzeichnet, daß** sie die im wesentlichen kupferfrei-modifizierten kupferbindenden Proteine enthält, die aus der Gruppe der blauen kupferbindenden Proteine ausgewählt sind, bestehend aus Auracyanin, Azurin, Phytocyanin, Plastocyanin, Rusticyanin, Ascorbatoxidase, Ceruloplasmin, Laccase, Nitritreduktase und Gemischen davon.

3. Zubereitung nach Anspruch 1, **dadurch gekennzeichnet, daß** die kupferbindenden Proteine in ihrer kupferfrei reduzierten Form aus der Gruppe ausgewählt sind, bestehend aus Cu, Zn-Superoxiddismutase, Dioxygenase, Monooxygenase, Aminoxidase, Diaminoxidase, Cytochrom-c-Oxidase, Galactoseoxidase, Lysyloxidase, Catecholoxidase, N₂O-Reduktase, Haemocyanin, Tyrosinase, Ubichinonoxidase und Gemischen davon.

4. Zubereitung nach Anspruch 1, **dadurch gekennzeichnet, daß** der Gehalt der modifizierten, im wesentlichen kupferfreien kupferbindenden Proteine im Bereich von 0,001 bis 1 Gew-% liegt, bezogen auf das Gesamtgewicht der Zubereitung.

5. Zubereitung nach Anspruch 1, **dadurch gekennzeichnet, daß** die Zubereitung zusätzlich hautaufhellende Mittel enthält, ausgewählt unter Bärentraubenblätterextrakt, Senfsamenextrakt von weißem Senf, Silberweidenrindenextrakt, Hibiskusblütenextrakt und Gemischen davon.

6. Zubereitung nach Anspruch 1, **dadurch gekennzeichnet, daß** die Zubereitung 0,1 bis 10 Gew-% eines gemahlenen, reinweißen Porzellans oder gemahlener weißer Perlen mit einer Teilchengröße im Bereich von 0,1 bis 100 µm enthält, bezogen auf das Gesamtgewicht der Zubereitung.

7. Zubereitung nach Anspruch 1, **dadurch gekennzeichnet, daß** die Zubereitung 0,1 bis 15 Gew-% reflektierende Pigmente enthält, ausgewählt aus der Gruppe, bestehend aus gemahlenen Glaspartikeln, TiO₂, ZnO, Perlmutt, Perglanzpigmente und Gemischen davon.

8. Zubereitung nach Anspruch 1, **dadurch gekennzeichnet, daß** die Zubereitung 1 bis 20 Gew-% eines organischen UV-Filters enthält.

9. Zubereitung nach Anspruch 1, **dadurch gekennzeichnet, daß** sie einen Extrakt von Glycyrrhiza glabra in einem Anteil von 0,1-6 Gew-% enthält, bezogen auf das Gewicht der Zubereitung.

10. Zubereitung nach Anspruch 1, **dadurch gekennzeichnet, daß** sie einen Komplex enthält, bestehend aus einem Extrakt von Glycyrrhiza glabra, Morus alba, Scutellaria baicalensis, Saxifraga stolonifera, Grapefruchtextrakt und Dinatriumethylendiamintetraessigsäure in einem Anteil von 0,1-6 Gew-%, bezogen auf das Gewicht der Zubereitung.

11. Kosmetische oder dermatologische Zubereitung mit Proteinen zur Hautaufhellung, **dadurch gekennzeichnet, daß** die Zubereitung einen wirksamen Gehalt an kupferfrei-modifizierten kupferbindenden Proteinen oder Enzymen aufweist, erhältlich durch Umsetzung von Enzymen oder Proteinen, die in der prosthetischen Gruppe Kupfer enthalten, mit einem kupferbindenden Chelatisierungsmittel oder durch Ionenaustausch an einem Kationenaustauscher, und Abtrennung der kupferfreien modifizierten Enzyme oder Proteine.

12. Verwendung von Proteinen oder Enzymen, denen Kupfer in der prosthetischen Gruppe durch eine Chelatisierungs- oder Kationenaustauschreaktion entzogen wurde, als Aufhellungsmittel für die Haut in kosmetischen Zubereitungen.

## Claims

1. A cosmetic or dermatological preparation containing proteins for skin lightening, which preparation comprises an effective amount of copper-free modified, copper-binding proteins or enzymes whose prosthetic group copper has been extracted by a prior exchange reaction, or which were produced without copper by recombinant methods.

2. A preparation according to claim 1, wherein said preparation contains the modified, i.e. substantially copper-free, copper-binding proteins selected from the group of the blue copper-binding proteins, which group consists of auracyanin, azurin, phytocyanin, plastocyanin, rusticyanin, ascorbate oxidase, ceruloplasmin, laccase, nitrite reductase and mixtures thereof.

3. A preparation according to claim 1, wherein the copper-binding proteins in their reduced, i.e. copper-free, form are selected form the group consisting of Cu, Zn superoxide dismutase, dioxygenase, monooxygenase, amine oxidase, diamine oxidase, cytochrome c oxidase, galactose oxidase, lysyl oxidase, catechol oxidase, N₂O reductase, haemocyanin, tyrosinase, ubiquinone oxidase and mixtures thereof.

4. A preparation according to claim 1, wherein the modified, substantially copper-free, copper-binding proteins make up 0.001 to 1% by weight, relative to the preparation's total weight.

5. A preparation according to claim 1, wherein said preparation additionally contains skin-whitening agents selected from among bearberry leave extract, mustard seed extract obtained from white mustard, white willow bark extract, hibiscus flower extract and mixtures thereof.

6. A preparation according to claim 1, wherein said preparation contains 0.1 to 10% by weight of a ground, pure white porcelain or of ground white pearls whose particle size ranges between 0.1 and 100 µm, relative to the preparation's total weight.

7. A preparation according to claim 1, wherein said preparation contains 0.1 to 15% by weight of reflective pigments selected from the group consisting of ground glass particles, TiO₂, ZnO, mother of pearl, pearlescent pigments and mixtures thereof.

8. A preparation according to claim 1, wherein said preparation contains 1 to 20% by weight of an organic UV filter.

9. A preparation according to claim 1, wherein said preparation contains an extract from Glycyrrhiza glabra in an amount ranging between 0.1 and 6% by weight, relative to the preparation's weight.

10. A preparation according to claim 1, wherein said preparation contains a complex consisting of extracts from Glycyrrhiza glabra, Morus alba, Scutellaria baicalensis, Saxifraga stolonifera, a grapefruit extract and disodium ethylenediamine tetraacetic acid, said complex making up 0.1 to 6% by weight, relative to the preparation's weight.

11. A cosmetic or dermatological preparation containing proteins for skin whitening, which preparation comprises an effective amount of copper-free modified, copper-binding proteins or enzymes obtainable by reacting enzymes or proteins containing copper in their prosthetic group with a copper-binding chelating agent, or by ion exchange using a cation exchanger, and separation of the copper-free, modified enzymes or proteins.

12. The use of proteins or enzymes whose prosthetic group copper has been extracted by a chelation reaction or a cation exchange reaction as a skin-whitening agent in cosmetic preparations.

## Revendications

1. Préparation cosmétique ou dermatologique aux protéines pour éclaircir la peau, **caractérisée en ce que** la préparation présente une teneur active en protéines ou enzymes liant le cuivre, modifiées sans cuivre, dont le cuivre dans le groupe prosthétique est extrait par une réaction d'échange préalable ou qui ont été préparées sans cuivre par un procédé de recombinaison.

2. Préparation selon la revendication 1, **caractérisée en ce qu'**elle contient les protéines liant le cuivre, essentiellement modifiées sans cuivre, qui sont choisies parmi le groupe des protéines liant le cuivre, bleues, consistant en l'auracyanine, l'azurine, la phytocyanine, la plastocyanine, la rusticyanine, l'ascorbate oxydase, la céruloplasmine, la laccase, la nitrite réductase et leurs mélanges.

3. Préparation selon la revendication 1, **caractérisée en ce que** les protéines liant le cuivre sont choisies sous leur forme réduite sans cuivre, parmi le groupe consistant en la Cu, Zn-superoxyde dismutase, la dioxygénase, la monooxygénase, l'aminoxydase, la diaminoxydase, la cytochrome C oxydase, la galactose oxydase, la lysyle oxydase, la catéchol oxydase, la NO₂ réductase, l'hémocyanine, la tyrosinase, l'ubiquinone oxydase et leurs mélanges.

4. Préparation selon la revendication 1, **caractérisée en ce que** la teneur en la protéine liant le cuivre, essentiellement exempte de cuivre, modifiée, se situe dans l'intervalle allant de 0,001 à 1% en poids, sur base du poids total de la préparation.

5. Préparation selon la revendication 1, **caractérisée en ce que** la préparation contient en outre, un agent éclaircissant la peau, choisi parmi l'extrait de feuilles de busserole, l'extrait de graine de moutarde blanche, l'extrait d'écorce de saule blanc, l'extrait de fleurs d'hibiscus et leurs mélanges.

6. Préparation selon la revendication 1, **caractérisée en ce que** la préparation contient 0,1 à 10% en poids d'une porcelaine broyée, blanc pur ou de perles blanches broyées, avec une taille des particules située dans l'intervalle allant de 0,1 à 100 µm, sur base du poids total de la préparation.

7. Préparation selon la revendication 1, **caractérisée en ce que** la préparation contient 0,1 à 15% en poids de pigments réfléchissants, choisis parmi le groupe consistant en des particules de verre broyées, le TiO₂, le ZnO, le nacre, des pigments nacrés et leurs mélanges.

8. Préparation selon la revendication 1, **caractérisée en ce que** la préparation contient 1 à 20% en poids d'un filtre organique contre les UV.

9. Préparation selon la revendication 1, **caractérisée en ce qu'**elle contient un extrait de *Glycyrrhiza glabra* en une quantité de 0,1-6% en poids, sur base du poids de la préparation.

10. Préparation selon la revendication 1, **caractérisée en ce qu'**elle contient un complexe consistant en un extrait de *Glycyrrhiza glabra,* de *Morus alba,* de *Scutellaria baicalensis,* de *Saxifraga stolonifera,* d'extrait de pamplemousse et de sel disodique de l'acide éthylènediaminetétraacétique en une quantité de 0,1-6% en poids, sur base du poids de la préparation.

11. Préparation cosmétique ou dermatologique aux protéines pour éclaircir la peau, **caractérisée en ce que** la préparation contient une teneur active en protéines ou enzymes liant le cuivre, modifiées sans cuivre, obtenues par transformation d'enzymes ou protéines, qui contiennent du cuivre dans le groupe prosthétique, avec un agent de chélation liant le cuivre ou par échange d'ions sur une échangeuse de cations, et séparation des enzymes ou protéines modifiées sans cuivre.

12. Utilisation de protéines ou enzymes, dont le cuivre dans le groupe prosthétique a été extrait par une réaction de chélation ou d'échange de cations, comme agent éclaircissant pour la peau dans des préparations cosmétiques.
